# EUROPEAN PATENT SPECIFICATION

(11) **EP 2 192 912 B1**
(45) Date of publication and mention of the grant of the patent: **01.06.2016**
(21) Application number: 08733387.8
(22) Date of filing: 22.04.2008
(51) Int. Cl.: A61P 33/10, A61P 1/00, A61P 19/06, A61K 36/61

(54) **ESSENTIAL OIL OF KUNZEA AMBIGUA AND METHODS OF USE**
ESSENTIELLES ÖL VON KUNZEA AMBIGUA UND ANWENDUNGSVERFAHREN
HUILE ESSENTIELLE DE KUNZEA AMBIGUA ET PROCÉDÉS D'UTILISATION

(30) Priority: 23.04.2007 AU 2007902118 P
(43) Date of publication of application: 09.06.2010
(73) Proprietor: Hood, John James David, Waterhouse TAS 7262 (AU)
(72) Inventor: Hood, John James David, Waterhouse TAS 7262 (AU)
(74) Representative: Evans, Huw David Duncan
(86) International application number: PCT/AU2008/000560
(87) International publication number: WO 2008/128295

(56) References cited:
- WO-A1-98/17749
- VEECH T: "Newsletter August 05", INTERNET CITATION, August 2005 (2005-08), XP008132031, Retrieved from the Internet: URL:http://www.naturalsolutionsbytia.com/u ploads/Newsletter%20August%2005.pd [retrieved on 2011-01-21]
- BLOOR S J: "ANTIVIRAL PHLOROGLUCINOLS FROM NEW ZEALAND KUNZEA SPECIES", JOURNAL OF NATURAL PRODUCTS, AMERICAN CHEMICAL SOCIETY, US, vol. 55, no. 1, 1 January 1992 (1992-01-01), pages 43-47, XP000905508, ISSN: 0163-3864, DOI: DOI:10.1021/NP50079A006
- LIS-BALCHIN M ET AL: "Pharmacological and antimicrobial studies on different tea-tree oils (Melaleuca alternifolia, Leptospermum scoparium or manuka and Kunzea ericoides or Kanuka), originating in Australia and New Zealand", MEDICINAL & AROMATIC PLANTS ABSTRACTS, SCIENTIFIC PUBLISHERS, SCIENTIFIC PUBLISHERS, NEW DELHI - INDIA, vol. 23, no. 2, 1 April 2001 (2001-04-01), XP018013444, ISSN: 0250-4367
- LIS-BALCHIN M ET AL: "BIOACTIVITY OF NEW ZEALAND MEDICINAL PLANT ESSENTIAL OILS", ACTA HORTICULTURAE, INTERNATIONAL SOCIETY FOR HORTICULTURAL SCIENCE, BE, no. 426, 1 January 1996 (1996-01-01), pages 13-30, XP002055778, ISSN: 0567-7572
- KHAMBAY B P S ET AL: "An insecticidal mixture of tetramethylcyclohexenedione isomers from Kunzea ambigua and Kunzea baxterii", PHYTOCHEMISTRY, PERGAMON PRESS, GB, vol. 59, no. 1, 1 January 2002 (2002-01-01), pages 69-71, XP004326317, ISSN: 0031-9422, DOI: DOI:10.1016/S0031-9422(01)00410-1
- ITO H ET AL: "Kunzeanones A, B, and C: novel alkylated phloroglucinol metabolites from Kunzea ambigua", TETRAHEDRON, ELSEVIER SCIENCE PUBLISHERS, AMSTERDAM, NL, vol. 60, no. 44, 25 October 2004 (2004-10-25), pages 9971-9976, XP004580198, ISSN: 0040-4020, DOI: DOI:10.1016/J.TET.2004.08.027
- KNIGHT G.: 'Essential OIls' KNOW YOURSELF, HEAL YOURSELF, [Online] 08 December 2006, pages 1 - 4, XP055204966 Retrieved from the Internet: <URL:http://www.web.archive.org/web/2006120 8112032/http://www.knowyourselfhealyourself .com/oils.shtml>
- VEECH T.: 'Newsletter August 05' NATURAL SOLUTIONS BY TIA, [Online] August 2005, XP008132031 Retrieved from the Internet: <URL:http://www.naturalsolutionsbytia.com/u ploads/Newsletter%20August%2005.pdf>
- HIDEYUKI I. ET AL.: 'dimeric Flavonol Glycoside and Galloylated C-Glucosylchromones from Kunzea ambigua' JOURNAL OF NATURAL PRODUCTS vol. 67, no. 3, 2004, pages 411 - 415, XP008132034

## Description

This invention relates to an improved medication.

Australian Patent 721156 entitled ESSENTIAL OIL AND METHODS OF USE describes an essential oil from the *Kunzea ambigua* and its topical use for the treatment of various conditions.

Further experimentation has shown unexpected results in the use of the oil in internal applications and the object of the present invention is to develop such use.

The invention includes the use of the essential oil from the *Kunzea ambigua* in internal applications for the treatment of medical conditions.

Specifically it has been found that the oil acts positively against parasitic infestation of humans and animals, and against certain other conditions, as set out in the claims.

Specifically, the present invention comprises a dosage regimen for internal administration, which comprises essential oil of *Kunzea ambigua,* in an amount of 2 to 15 drops, for administration and treatment of various conditions as defined in claim 1. Preferred dosage regimens are defined in the remaining claims.

In order that the invention may be more readily understood, applications of the invention and basis therefor will now be described.

A first patient was suffering substantial weight loss over a relatively short period (from 95Kg to 88Kg)

The patient was originally suspected of having a thyroid malfunction, but tests did not show more than a minor irregularity and a second diagnoses was lung cancer (although this was not indicated by X-ray).

A second opinion was sought and the medical practitioner did certain tests and weighed the patient and asked him to return for the test results after a short period.

During this period, the patient self medicated and settled on taking two doses a day of seven to eight drops of the *Kunzea ambigua* oil in 16mL of water, gargled and then swallowed.

The results were dramatic, the patient gained weight at a rate of some 0.5 Kg per day.

It is the patient's belief that he was, in fact, infested with parasites and the kunzea ambigua oil killed the infestation and enabled his digestive system to operate normally. The treatment was discontinued after approximately one week on the assumption that the weight gain indicated that the cause of the loss had been removed. After several years the problem has not reoccurred.

It is believed that the oil acted as a highly effective anthelmintic.

A middle aged man with irritable bowel syndrome self medicated with 4 drops each of *Kunzea ambigua* and Fragonia *(Agonis fragrans*) oils three times a day with food. Within a week, the symptoms of the disease disappeared.

A 65 year old man had severe gout in the fingers and wrist. External application in these areas gives good results but is slow as the passage of oil through the skin in these areas is slow. He took 5-6 drops of *Kunzea ambigua* in water or fruit juice each morning and in 11 days the swelling had regressed and the pain was substantially relieved. The client in this case also took conventional pain killers during the early part of the treatment but after five days could be stopped.

The oil has been laboratory tested against viruses and it has shown a very high kill rate. It is believed that the oil is efficacious against many diseases caused by internal germs and viruses.

The oil can also be successfully used against parasite attacks in animals.

In Europe, and specifically in France, essential oils are used by mainstream doctors, are prescribed by them for treating certain diseases, the prescriptions are made up by pharmacists, are used internally in many applications and are used at higher rates than have generally been the case in Britain, Australia and the United States of America.

Trials of the use of *kunzea oil* have been carried out in France by Dr Daniel Penoel, a leading practitioner in this field. These trials have been clinically based and have been done without external funding.

Dr Penoil states
'Using *kunzea ambigua* in diluted form and in external applications can only reveal some possibilities. Obviously using higher concentrations and using it internally opens a completely new area of applications. .... What was done with *kunzea ambigua* in this medical context confirmed that it has a very strong power.'

As a result of these trials, Dr Penoel has reported:
*Kunzea ambigua* taken internally has shown a very strong action on a certain number of viral conditions
   1. *kunzea ambigua* has shown a strong effect on the cleansing of arteries that were partially clogged by atheromatosis plaques
   2. *kunzea ambigua* has shown a powerful action on degenerative conditions involving certain forms of cancers (veterinary medicine).
   3. *kunzea ambigua* has shown a very strong action on inflammatory processes, including inflammation linked with auto-immune diseases, in particular Crohn's disease.
   4. *kunzea ambigua* has shown a very strong action, for some receptive patients, on the psyche, to give strength and resilience.
   5. *kunzea ambigua* has shown a very strong action on consequences of chronic viral infections, like EBV (glandular fever), with results that no pharmaceutical synthetic molecule can achieve.
   *6. Kunzea ambigua* has shown a positive action on the liver function.

Dr Penoel has also carried out trials on animals and has reported:
"In veterinary medicine, it is easy to understand that many treatments can be given that would not be possible in human medicine. Several cases of dogs that should 'normally' have died are still living and *Kunzea ambigua* played a major role in rescuing them. Other animals have been treated with excellent results that were found amazing by the veterinary surgeons.

If one understands that *Kunzea ambigua* has a multiple facet action on several aspects of physiopathological processes that underlie many diseases, it is clear that the list of diseases in which it may exert a positive role is extremely vast."

## Claims

1. A dosage regimen comprising essential oil of *Kunzea ambigua,* for internal administration, in an amount of 2 to 15 drops, for use as an anthelmintic in the treatment of parasite infestation, or for use in the treatment of gout or irritable bowel syndrome, the dosage regimen being:
2 to 4 times daily by swallowing for use as an anthelmintic in the treatment of parasitic infestation;
up to 4 times daily in water or fruit juice for use in the treatment of gout; or
2 to 4 times daily with food for use in the treatment of irritable bowel syndrome.

2. A dosage regimen according to claim 1 for use as an anthelmintic in the treatment of parasitic infestation, comprising 7 to 8 drops of essential oil of *Kunzea ambigua* in water, for swallowing in two doses per day.

3. A dosage regimen according to claim 1 for use in the treatment of gout, comprising 5 to 6 drops of essential oil of *Kunzea ambigua* taken daily in water or fruit juice.

4. A dosage regimen according to claim 1 for use in the treatment of irritable bowel syndrome, comprising 4 drops each of essential oil of *Kunzea ambigua* and *Agonis fragrans* for administration three times per day with food.

## Patentansprüche

1. Dosierungsschema, umfassend ätherisches Öl von *Kunzea ambigua,* zur innerlichen Verabreichung in einer Menge von 2 bis 15 Tropfen zur Verwendung als Anthelminthikum in der Behandlung von Parasitenbefall oder zur Verwendung in der Behandlung von Gicht oder Reizdarm, wobei das Dosierungsschema wie folgt vorgesehen ist:
2- bis 4-mal täglich durch Schlucken zur Verwendung als Anthelminthikum in der Behandlung von Parasitenbefall;
bis zu 4-mal täglich in Wasser oder Obstsaft zur Verwendung in der Behandlung von Gicht; oder
2- bis 4-mal täglich mit Nahrung zur Verwendung in der Behandlung von Reizdarm.

2. Dosierungsschema nach Anspruch 1 zur Verwendung als Anthelminthikum in der Behandlung von Parasitenbefall, umfassend 7 bis 8 Tropfen ätherisches Öl von *Kunzea ambigua* in Wasser zum Schlucken in zwei Dosen pro Tag.

3. Dosierungsschema nach Anspruch 1 zur Verwendung in der Behandlung von Gicht, umfassend 5 bis 6 Tropfen ätherisches Öl von *Kunzea ambigua* bei täglicher Einnahme in Wasser oder Obstsaft.

4. Dosierungsschema nach Anspruch 1 zur Verwendung in der Behandlung von Gicht, umfassend jeweils 4 Tropfen ätherisches Öl von *Kunzea ambigua* und *Agonis fragrans* zur dreimal täglichen Verabreichung mit Nahrung.

## Revendications

1. Régime posologique comprenant l'huile essentielle de *Kunzea ambigua,* pour une administration interne, dans une quantité comprise entre 2 et 15 gouttes, pour une utilisation en tant qu'un anthelmintique dans le traitement d'une infestation parasitaire, ou pour une utilisation dans le traitement de la goutte ou du syndrome du côlon irritable, le régime posologique étant :
de 2 à 4 fois par jour en avalant pour une utilisation en tant qu'un anthelmintique dans le traitement d'une infestation parasitaire ;
jusqu'a 4 fois par jour dans de l'eau ou dans un jus de fruit dans le traitement de la goutte ; ou
de 2 à 4 fois par jour avec de la nourriture dans le traitement du syndrome du côlon irritable.

2. Régime posologique selon la revendication 1 pour une utilisation en tant qu'un anthelmintique dans le traitement d'une infestation parasitaire, comprenant de 7 à 8 gouttes d'huile essentielle de *Kunzea ambigua* dans de l'eau, pour avaler en deux doses par jour.

3. Régime posologique selon la revendication 1 pour une utilisation dans le traitement de la goutte, comprenant de 5 à 6 gouttes d'huile essentielle de *Kunzea ambigua* prises quotidiennement dans de l'eau ou dans un jus de fruit.

4. Régime posologique selon la revendication 1 pour une utilisation dans le traitement du syndrome du côlon irritable, comprenant 4 gouttes d'huile essentielle de *Kunzea ambigua et* de *Agonis fragrans* pour une administration trois fois par jour avec de la nourriture.
